# EUROPEAN PATENT APPLICATION

(11) **EP 2 319 503 A2**
(43) Date of publication of application: **11.05.2011**
(21) Application number: 09755059.4
(22) Date of filing: 29.05.2009
(51) Int. Cl.: A61K 9/22, A61K 9/20, A61K 47/48, A61K 31/496, A61K 31/573, A61K 9/14

(54) **DRUG DELIVERY CARRIER**

(30) Priority: 29.05.2008 KR 20080050271
(71) Applicant: Kwon, Soon-Chang, Seoul 121-090 (KR); Nam, Jeong Sun, Gyeonggi-do 448-170 (KR)
(72) Inventor: KWON, Soon-Chang, Mapo-gu, Seoul 121-090 (KR)
(74) Representative: Cabinet Plasseraud
(86) International application number: PCT/KR2009/002886
(87) International publication number: WO 2009/145594

(57) **Abstract**

The present disclosure relates to a drug delivery carrier including: (a) a biocompatible polymer; and (b) a hydrophobic group conjugated to the polymer. The drug delivery carrier according to the present disclosure having the hydrophobic group conjugated to the biocompatible polymer may be useful for adsorption of synthetic drugs having very low solubility in water. Further, it may regulate discharge rate of adsorbed drugs by regulating a portion of hydrophobic groups conjugated to the polymeric material. Thus, the present disclosure provides a broad-spectrum platform technology applicable to new hydrophobic synthetic drugs to be developed in the future as well as those that have been developed already but face difficulties due to low bioavailability. The disclosed drug delivery carrier may provide considerable therapeutic convenience for patients by combining stained-release characteristics with the ability for adsorption of a hydrophobic drug having low bioavailability.

The drug delivery carrier according to the present disclosure may also be applied to protein therapeutics. For patent-expired first-generation protein drugs requiring daily or once-in-two-or-three-days injection, the present disclosure improves convenience by allowing second-generation injection formulations that are administered once a week or once or twice a month. As used herein, a "first-generation protein drug" refers to a biomedicine based on a natural protein prepared by a gene recombination technique and a "second-generation protein drug" refers to a biopharmaceutical improvement of a first-generation protein drug through formulation or modification of molecular structure for increasing half-life or extending treatment period through sustained release. The present disclosure provides a strong tool capable of achieving the desired effect simply by mixing with or adsorbing to the drug delivery carrier, unlike known techniques requiring modification or introduction of a special molecular structure to the first-generation or second-generation protein drug. Thus, application of the disclosed drug delivery carrier will shorten development time of next-generation protein drugs and will effectively contribute to increasing use of hydrophobic synthetic drugs. Ultimately, the disclosed drug delivery carrier will be useful for development of competitive new medicines such as sustained-release proteins and synthetic pharmaceuticals.

## Description

### FIELD OF THE INVENTION

The present disclosure relates to a drug delivery carrier. More particularly, the disclosure relates to a drug delivery carrier for carrying proteins, peptides and hydrophobic drugs.

### BACKGROUD OF THE INVENTION

In general, synthetic drugs, particularly hydrophobic synthetic drug having extremely low solubility in water, have very good medicinal effect, but very poor bioavailability. Thus, there have been many efforts in the pharmaceutical industry and in order to effectively deliver them while improving their bioavailability.

For example, paclitaxel, which was discovered by the Research Triangle Institute (RTI) in 1967, is used to treat lung cancer, breast cancer, ovarian cancer and advanced forms of Kaposi's sarcoma and is the representative anticancer drug given FDA approval in 1992. Also well-known as its tradename TAXOL, this anticancer drug is a natural substance found in the bark of the Pacific yew tree. It inhibits cancer growth by binding to ß-tubulin of cancer cells and interfering with binding and breakdown of microtubules in the cancer cells. Despite its superior anticancer activity, it is difficult to prepare paclitaxel into injection formulation because of extremely low solubility in water (0.3 g/mL).

To overcome this problem, a new formulation in which paclitaxel is dispersed in a 50:50 mixture of Cremophor EL and ethanol was developed, which is then diluted with physiological saline and administered intravenously. However, paclitaxel diluted with aqueous solution is physically stable for only 12 to 24 hours. Over prolonged time, it precipitates after all, resulting in very low bioavailability. As such, the high hydrophobicity of some synthetic drugs including anticancer drugs is the major hindrance to the successful development of medicines *(*Biomolecules 8: 202-208(2007)).

Meanwhile, for protein drugs which are relatively more water-soluble than the synthetic drugs such as the hydrophobic anticancer drug, the introduction of the drug delivery carrier is focused on improvement of patient convenience rather than increase of solubility in water. That is to say, user convenience may be improved if the drugs can be administered once a week or once or twice a month rather than every day or once in 2 to 3 days. Further, it is also important to suppress initial burst of drugs immediately after the injection in order to minimize undesired side effects.

In this regard, several new techniques have been developed recently. They include: 1) partial modification of an amino acid sequence of the protein drug, 2) extension of in vivo half-life of the protein drug by conjugation with polyethylene glycol (PEG) or the Fc region of an antibody, 3) ensuring sustained release through crystallization of protein, 4) realizing sustained release of the protein drug through a new formulation, and 5) nasal or buccal mucosal administration or oral administration.

To achieve these purposes, efforts are continuously made to apply polymeric materials having superior biodegradability or biocompatibility to drug delivery.

All the disclosure of the literatures and patents cited in the description is hereby incorporated by reference in its entirety.

### DETAILED DESCRIPTION OF THIS INVETNION

### Technical Problem

The inventors of the present disclosure have made efforts to develop a drug delivery carrier capable of improving bioavailability of hydrophobic synthetic drugs having low solubility in water and, at the same time, very stably delivering water-soluble protein drugs. In particular, they aimed at developing a drug delivery carrier capable of reducing the frequency of injection of drugs that need to be injected frequently for therapeutic purposes and inducing long-lasting sustained release of the drugs. They have confirmed that a drug delivery carrier prepared by introducing a hydrophobic group to a biocompatible polymer can adsorb a drug at high efficiency and enables sustained release of the drug.

The present disclosure is directed to providing a drug delivery carrier.

Other features and aspects will be apparent from the following detailed description, the drawings, and the claims.

### Technical Solution

In one general aspect, the present disclosure provides a drug delivery carrier including:
(a) a biocompatible polymer; and (b) a hydrophobic group conjugated to the polymer.

The biocompatible polymer may be any biocompatible polymer commonly used in the art.

Specifically, the biocompatible polymer may be a synthetic polymer or a natural polymer.

According to a specific embodiment of the present disclosure, the synthetic polymer as the biocompatible polymer may be polyester, polyhydroxyalkanoate (PHA), poly(α-hydroxy acid), poly(p-hydroxy acid), poly(3-hydroxybutyrate-co-valerate) (PHBV), poly(3-hydroxypropionate) (PHP), poly(3-hydroxyhexanoate (PHH), poly(4-hydroxy acid), poly(4-hydraxybutyrate), poly(4-hydroxyvalerate), poly(4-hydroxyhexanoate), poly(esteramide), polycaprolactone, polylactide, polyglycolide, poly(lactide-co-glycolide) (PLGA), polydioxanone, polyorthoester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHA-PEG, ethylene vinyl alcohol copolymer (EVOH), polyurethane, silicone, polyester, polyolefin, polyisobutylene, ethylene-α-olefin copolymer, styrene-isobutylene-styrene triblock copolymer, acryl polymer or copolymer, vinyl halide polymer or copolymer, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatic, polystyrene, polyvinyl ester, polyvinyl acetate, ethylene-methyl methacrylate copolymer, acrylonitrile-styrene copolymer, ABS resin, ethylene-vinyl acetate copolymer, polyamide, alkyd resin, polyoxymethylene, polyimide, polyether, polyacrylate, polymethacrylate or polyacrylic acid-co-maleic acid.

According to a specific embodiment of the present disclosure, the synthetic polymer as the biocompatible polymer may be a biodegradable/biocompatible polymer, including polyester, polyhydroxyalkanoate (PHA), poly(α-hydroxy acid), poly(β-hydroxy acid), poly(3-hydroxybutyrate-co-valerate) (PHBV), poly(3-hydroxypropionate) (PHP), poly(3-hydroxyhexanoate) (PHH), poly(4-hydroxy acid), poly(4-hydroxybutyrate), poly(4-hydroxyvalerate), poly(4-hydroxyhexanoate), poly(esteramide), polycaprolactone, polylactide, polyglycolide, poly(lactide-co-glycolide) (PLGA), polydioxanone, polyorthoester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene or PHA-PEG.

According to a specific embodiment of the present disclosure, the natural polymer as the biocompatible polymer may be chitosan, dextran, cellulose, heparin, hyaluronic acid, alginate, inulin, starch or glycogen, more specifically, chitosan or cellulose, most specifically, chitosan.

As used herein, the terms used to express the biocompatible polymers include their derivatives. For example, the terms "cellulose" and "dextran" respectively include their derivatives carboxymethyl cellulose and carboxymethyl dextran.

The biocompatible polymer used in the present disclosure needs not have a particularly limited molecular weight. Specifically, it may have an average molecular weight 1,000 kDa or smaller, more specifically 300 kDa or smaller, most specifically 100 kDa or smaller. The polymeric material may be selected considering its characteristics, the protein desired to be adsorbed thereto, the properties of the corresponding synthetic drug, or the like.

According to the present disclosure, a hydrophobic group is conjugated to the biocompatible polymer to prepare the drug delivery carrier.

The hydrophobic group is not specially limited. Specifically, it may be an aliphatic compound or aromatic compound having 4 or more carbon atoms.

The aliphatic compound having 4 or more carbon atoms as the hydrophobic group may be, specifically an aliphatic compound having 5 or more carbon atoms, more specifically an aliphatic compound having 5 to 30 carbon atoms, most specifically an aliphatic compound having 5 to 20 carbon atoms.

According to a specific embodiment of the present disclosure, the hydrophobic group may be an aromatic compound having 1 to 3 (specifically 1 or 2) phenyl group(s).

According to a specific embodiment of the present disclosure, the hydrophobic group conjugated to the polymer is alkyl having 4 or more carbon atoms, alkenyl having 4 or more carbon atoms, cycloalkyl having 3 or more carbon atoms, alkoxy having 4 or more carbon atoms, aryl, carboxyaryl, aryl phosphate, arylamine, heteroaryl, arylalkyl, arylalkenyl, or alkylaryl.

The term "alkyl" refers to a linear or branched saturated hydrocarbon group having a designated number of carbon atoms. The term "alkenyl" refers to a linear or branched unsaturated hydrocarbon group having a designated number of carbon atoms. The term "cycloalkyl" refers to a cyclic hydrocarbon radical having a designated number of carbon atoms. Specifically, it may be "C₃-C₈ cycloalkyl" and includes cyclopropyl, cyclobutyl and cyclopentyl. The term, "alkoxy" refers to an -O-alkyl, group.

The term "aryl" refers to a fully or partially unsaturated, substituted or unsubstituted monocyclic or polycyclic carbon ring, such as monoaryl or biaryl. The monoaryl may have 5 or 6 carbon atoms, and the biaryl may have 9 or 10 carbon atoms. Most specifically, the aryl may be substituted or unsubstituted phenyl. A monoaryl, e.g., phenyl, may be substituted with various substituents at various positions. For example, it may be substituted with a halo, hydroxyl, nitro, cyano, C₁-C₄ substituted or unsubstituted linear or branched alkyl, C₁-C₄linear or branched alkoxy, alkyl-substituted sulfanyl, phenoxy, C₃-C₆ cycloheteroalkyl, or substituted or unsubstituted amino group. A biaryl, e.g., biphenyl (diphenyl) or naphthyl, may be substituted with various substituents at various positions. Specifically, it may be substituted with a halo, hydroxyl, nitro, cyano, C₁-C₄ substituted or unsubstituted linear or branched alkyl, C₁-C₄linear or branched alkoxy, or substituted or unsubstituted amino group. More specifically, it may be substituted with an alkyl-substituted amino group.

The term "heteroaryl" refers to a heterocyclic aromatic group containing N, O or S as heteroatom(s). Specifically, the heteroaryl may contain N as a heteroatom.

The term "arylalkyl (aralkyl)" refers to an aryl group attached to one or more alkyl group(s). Specifically, it may be a benzyl group. The term "alkylaryl" refers to an alkyl group attached to one or more aryl group(s). The term "arylalkenyl" refers to an aryl group attached to one or more alkenyl group(s), for example, phenylethenyl.

More specifically, the hydrophobic group conjugated to the polymer may be aryl, carboxyaryl, aryl phosphate, arylamine, heteroaryl, arylalkyl, arylalkenyl or alkylaryl. Further more specifically, it may be aryl, and most specifically, it may be monoaryl or biaryl.

In the drug delivery carrier according to the present disclosure, the biocompatible polymer may have an amino group, the hydrophobic group may be amide-bonded to the amino group, and the drug delivery carrier may be represented by Chemical Formula I:

R₁-NH-CO-R₂ **(1)**

wherein R₁ is the backbone of the biocompatible polymer; and R₂ is aryl, heteroaryl, arylalkyl, arylalkenyl or alkylaryl.

In Chemical Formula I, the amino group conjugated to the biocompatible polymer may originate from the polymer or from other substance (e.g., a linker). And, the C=O moiety of the hydrophobic group may originate from the hydrophobic group or from other substance (e.g., a linker).

Alternatively, the biocompatible polymer may have a carboxyl group, the hydrophobic group may be amide-bonded to the carboxyl group, and the drug delivery carrier may be represented by Chemical Formula II:

R₁-CO-NH-R₂ **(2)**

wherein R₁ is the backbone of the biocompatible polymer; and R₂ is aryl, heteroaryl, arylalkyl, arylalkenyl or alkylaryl.

In Chemical Formula II, the N-H group conjugated to the biocompatible polymer may originate from the hydrophobic group or from other substance (e.g., a linker). And, the amino group of the hydrophobic group may originate from the biocompatible polymer or from other substance (e.g., a linker).

Some specific embodiments of the present disclosure will be described referring to Reaction Scheme 1:

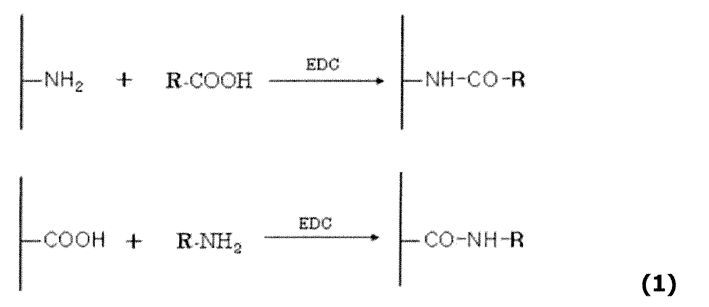

A cationic (having amino group) biocompatible natural polymer that may be used to prepare the drug delivery carrier of the present disclosure includes chitosan and chitooligosaccharides.

The hydrophobic group conjugated to the polymer may be a donor having a monophenyl or diphenyl (C₆H₅-C₆H₅) group. The selection of the hydrophobic group and the conjugation thereof may be varied depending on the structural characteristics of the polymer, the properties of the drug to be adsorbed, the properties of the finally prepared drug delivery carrier, or the like (see Reaction Scheme 1).

A hydrophobic group suitable to be conjugated to the polymeric material having an amino group may be benzoic acid, diphenyl phosphate, 3,3-diphenylpropionic acid, 2-phenylacetic acid, diphenylacetic acid, or the like.

A hydrophobic group suitable to be conjugated to the polymer having a carboxyl group may be 2,2-diphenylethylamine, 1,2-diphenylethylamine, 3,3-diphenylpropylamine, 2-aminobiphenyl, aminodiphenylmethane, benzylamine, diphenylamine, N-phenylbenzylamine, or the like.

A carbodiimide may be used to conjugate the hydrophobic group to the polymeric material. The carbodiimide used for the conjugation may be a water-soluble substance such as 1-ethyl-3-(3-dimethylaminopropyl)carbodiimide hydrochloride (EDC or EDAC), EDC/sulfo-N-hydroxysulfosuccinimide (NHS) or 1-cyclohexyl-3-(2-morpholinoethyl)carbodiimide (CMC), a water-insoluble substance such as dicyclohexylcarbodiimide (DCC) or diisopropylcarbodiimide (DIC), or an appropriate combination of the water-soluble and water-insoluble substances.

As described, the hydrophobic group that is conjugated to the polymer chain is selected from a material which does not cause severe in vivo toxicity when released as the polymer is degraded or the conjugated part is hydrolyzed.

Specifically, the hydrophobic group may be conjugated to the polymeric material at a concentration of 10 to 300 mM, more specifically 20 to 200 mM.

The drug delivery carrier of the present disclosure is particularly adequate for carrying proteins, peptides and non-hydrophilic chemical drugs.

The protein or peptide carried by the drug delivery carrier of the present disclosure is not particularly limited. They include hormones, hormone analogues, enzymes, enzyme inhibitors, signaling proteins or parts thereof, antibodies or parts thereof, single-chain antibodies, binding proteins or binding domains thereof, antigens, adhesion proteins, structural proteins, regulatory proteins, toxic proteins, cytokines, transcription factors, blood clotting factors, vaccines, or the like, but are not limited thereto. More specifically, the protein or peptide carried by the drug delivery carrier of the present disclosure may be insulin, insulin-like growth factor 1 (IGF-1), growth hormone, erythropoietin, granulocyte-colony stimulating factor (G-CSF), granulocyte-macrophage colony stimulating-factor (GM-CSF), interferon α, interferon β, interferon y, interleukin-1 α and β, interleukin-3, interleukin-4, interleukin-6, interleukin-2, epidermal growth factor (EGF), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosine α₁, triptorelin, bivalirudin, carbetocin, cyclosporin, exidine, lanreotide, luteinizing hormone-releasing hormone (LHRH), nafarelin, parathyroid hormone, pramlintide, enfuvirtide (T-20), thymalfasin or ziconotide.

The protein or peptide carried by the drug delivery carrier of the present disclosure is not particularly limited. It may be, for example, acivicin, aclarubicin, acodazole, acrisorcin, adozelesin, alanosine, aldesleukin, allopurinol sodium, altretamine, aminoglutethimide, amonafide, ampligen, amsacrine, androgens, anguidine, aphidicolin glycinate, asaley, asparaginase, 5-azacitidine, azathioprine, Bacillus Calmette-Guerin (BCG), Baker's antifol, β-2-deoxythioguanosine, bisantrene HCl, bleomycin sulfate, busulfan, buthionine sulfoximine, BWA 773U82, BW 502U83·HCl, BW 7U85 mesylate, ceracemide, carbetimer, carboplatin, carmustine, chlorambucil, chloroquinoxaline sulfonamide, chlorozotocin, chromomycin A3, cisplatin, cladribine, corticosteroids, Corynebacterium parvum, CPT-11, crisnatol, cyclocytidine, cyclophosphamide, cytarabine, cytembena, dabis maleate, dacarbazine, dactinomycin, daunorubicin HCl, deazauridine, dexrazoxane, dianhydrogalactitol, diaziquone, dibromodulcitol, didemnin B, diethyldithiocarbamate, diglycoaldehyde, dihydro-5-azacytidine, doxorubicin, echinomycin, edatrexate, edelfosine, eflornithine, Elliott's solution, elsamitrucin, epirubicin, esorubicin, estramustine phosphate, estrogens, etanidazole, ethiofos, etoposide, fadrazole, fazarabine, fenretinide, filgrastim, finasteride, flavone acetic acid, floxuridine, fludarabine phosphate, 5-fluorouracil, Fluosol™, flutamide, gallium nitrate, gemcitabine, goserelin acetate, hepsulfam, hexamethylene bisacetamide, homoharringtonine, hydrazine sulfate, 4-hydroxyandrostenedione, hydrozyurea, idarubicin HCl, ifosfamide, 4-ipomeanol, iproplatin, isotretinoin, leucovorin calcium, leuprolide acetate, levamisole, lomustine, lonidamine, maytansine, mechlorethamine hydrochloride, melphalan, menogaril, merbarone, 6-mercaptopurine, mesna, methanol extraction residue of Bacillus Calmette-Guerin, methotrexate, N-methylformamide, mifepristone, mitoguazone, mitomycin-C, mitotane, mitoxantrone hydrochloride, monocyte/macrophage colony-stimulating factor, nabilone, nafoxidine, neocarzinostatin, octreotide acetate, ormaplatin, oxaliplatin, paclitaxel, pala, pentostatin, piperazinedione, pipobroman, pirarubicin, piritrexim, piroxantrone hydrochloride, PIXY-321, plicamycin, porfimer sodium, prednimustine, procarbazine, progestins, pyrazofurin, razoxane, sargramostim, semustine, spirogermanium, spiromustine, streptonigrin, streptozocin, sulofenur, suramin sodium, tamoxifen, taxotere, tegafur, teniposide, terephthalamidine, teroxirone, thioguanine, thiotepa, thymidine injection, tiazofurin, topotecan, toremifene, tretinoin, trifluoperazine hydrochloride, trifluridine, trimetrexate, uracil mustard, vinblastine sulfate, vincristine sulfate, vindesine, vinorelbine, vinzolidine, Yoshi 864 or zorubicin.

According to a specific embodiment of the present disclosure, the conjugation between the biocompatible polymer and the hydrophobic group is mediated by a linker.

The linker may be any compound used in the art. A suitable linker may be considering the functional group(s) of the corresponding protein or peptide. For example, the linker may include N-succinimidyl iodoacetate, N-hydroxysuccinimidyl bromoacetate, m-maleimidobenzoyl-N-hydroxysuccinimide ester, m-maleimidobenzoyl-N-hydroxysulfosuccinimide ester, N-maleimidobutyryloxysuccinamide ester, N-maleimidobutyryloxysulfosuccinamide ester, E-maleimidocaproic acid hydrazide·HCl, N-(E-maleimidocaproyloxy)-succinamide, N-(E-maleimidocaproyloxy)-sulfosuccinamide, maleimidopropionic acid N-hydroxysuccinimide ester, maleimidopropionic acid N-hydroxysulfosuccinimide ester, maleimidopropionic acid hydrazide·HCl, N-succinimidyl-3-(2-pyridyldithio)propionate, N-succinimidyl-4-(iodoacetyl)aminobenzoate, succinimidyl-(N-maleimidomethyl)cyclohexane-1-carboxylate succinimidyl-4-(p-maleimidaphenyl)butyrate, sulfosuccinimidyl-(4-iodoacetyl)aminobenzoate, sulfosuccinimidyl-4-(N-maleimidomethyl)cyclohexane-1-carboxylate, sulfosuccinimidyl-4-(p-maleimidophenyl)butyrate, m-maleimidobenzoic acid hydrazide·HCl, 4-(N-maleimidomethyl)cyclohexane-1-carboxylic acid hydrazide·HCl, 4-(4-N-maleimidophenyl)butyric acid hydrazide·HCl, N-succinimidyl 3-(2-pyridyldithio)propionate, bis(sulfosuccinimidyl)suberate, 1,2-di[3'-(2'-pyridyldithio)propionamido]butane, disuccinimidyl suberate, dissuccinimidyl tartarate), disulfosuccinimidyl tartarate, dithio-bis(succinimidyl propionate), 3,3'-dithio-bis(sulfasuccinimidyl propionate), ethylene glycol bis(succinimidylsuccinate and ethylene glycol bis(sulfosuccinimidylsuccinate), but is not limited thereto.

An example of using a linker according to the present disclosure is illustrated by Reaction Scheme 2:

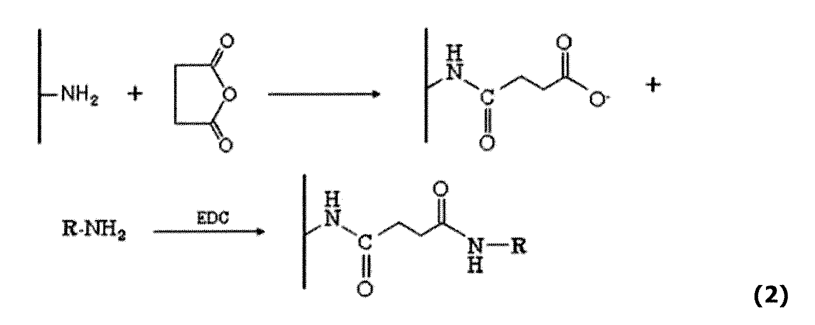

The drug delivery carrier according to the present disclosure may be prepared by directly conjugating the hydrophobic group chemically to the polymeric material having a carboxyl group or an amino group using a carbodiimide as a zero-length cross-linker. Alternatively, the functional group of the polymer may be modified to improve the applicability of the polymeric material. For example, a chitosan polymer having an amino group may be conjugated using an appropriate spacer or linker so as to modify the amino group of chitosan with a terminal carboxyl group (see Reaction Scheme 2). Reversely, carboxymethyl cellulose having a carboxyl group may be conjugated using an appropriate spacer or linker to prepare carboxymethyl cellulose having a terminal amino group.

According to a specific embodiment of the present disclosure, the drug delivery carrier of the present disclosure releases the drug in a sustained manner.

According to a specific embodiment of the present disclosure, 0.5-20 parts by weight, more specifically 1-12 parts by weight, of the hydrophobic group is conjugated to the biocompatible polymer based on 1 part by weight of the polymer.

According to a specific embodiment of the present disclosure, the drug delivery carrier of the present disclosure is capable of regulating sustained release of a drug depending on: (i) the kind of the hydrophobic group conjugated to the biocompatible polymer; (ii) the amount of the hydrophobic group conjugated to the biocompatible polymer; (iii) the content of the drug delivery carrier for adsorbing the drug; or a combination thereof.

The drug delivery carrier of the present disclosure may be prepared into a pharmaceutical composition. In this case, the pharmaceutical composition may comprise a pharmaceutically acceptable excipient. The pharmaceutically acceptable excipient may be one commonly used in the art. It may include lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, mineral oil, or the like, but is not limited thereto. In addition to the aforesaid components, the pharmaceutical composition may further comprise a lubricant, wetting agent, sweetener, flavor, emulsifier, suspending agent, preservative, or the like. Appropriate pharmaceutically acceptable excipients and formulations are described in detail in Remington's Pharmaceutical Sciences (19th ed., 1995).

An adequate administration dose of the pharmaceutical composition may be varied depending on such factors as the method of formulation, administration method, age, body weight, sex and pathological condition of the patient, diet, administration time, administration route, excretion rate and response sensitivity.

The pharmaceutical composition may be formulated into single or multiple dosage forms according to a method known to those skilled in the art using a pharmaceutically acceptable excipient and/or vehicle. The formulation may be in the form of oil, solution in aqueous medium, suspension, emulsion, extract, powder, granule, tablet or capsule, and may further include a dispersant or stabilizer.

The hydrophobic group conjugated to the polymer chain according to the present disclosure, e.g. a mono- or diphenyl group, is conjugated to low-molecular-weight synthetic pharmaceuticals or protein drugs (biopharmaceuticals) based on hydrophobic interactions. As a result, adsorption of the drugs having low solubility in water and thus their delivery into the body may be greatly improved. At the same time, the hydrophobic groups widely conjugated throughout the polymer chain significantly improve the dispersibility of the drug, such that the drug may be uniformly adsorbed to the polymeric material.

In addition to the improved adsorption between the polymeric material and the drug and the improved dispersion, a secondary effect, i.e. sustained release of the drug adsorbed to the drug delivery carrier, is attained. This is because the polymeric material is decomposed very slowly in the body. As the biocompatible polymer is decomposed by enzymes, the drug adsorbed to the polymer chain is released slowly over time in a sustained manner.

Since there are various biocompatible natural or synthetic polymers and various hydrophobic groups that can be used to prepare the drug delivery carrier according to the present disclosure and the drug delivery carriers resulting from various combinations thereof have varying physical and chemical properties, a desired drug delivery carrier may be prepared depending on purposes.

### BRIEF DESCRIPTION OF THE DRAWINGS

Fig. 1 schematically illustrates a conjugation reaction between a polymeric material having an amino group or a polymeric material having an carboxyl group and a hydrophobic group mediated by 1-ethyl-3-(3-dimethylaminopropyl)carbodiimide (EDC);
Fig. 2 schematically illustrates a process whereby a polymeric material having an amino group is conjugated using a linker to modify the amino group of the polymeric material with a terminal carboxyl group and the polymeric material with the terminal carboxyl group is conjugated with a hydrophobic group by EDC;
Fig. 3 shows an SDS-PAGE result showing the protein-adsorbing capacity of a chitosan-benzoic acid conjugate prepared from conjugation with 15 mM benzoic acid (In the figure, bands 1-5 show the protein-adsorbing capacity of the chitosan-benzoic acid conjugate, and bands 6-10 show the protein-adsorbing capacity of chitosan.);
Fig. 4 shows an in vitro release pattern of bovine serum albumin (BSA) when a chitosan-benzoic acid conjugate prepared from conjugation with 25 mM benzoic acid was used;
Fig. 5 shows a pharmacokinetic pattern of granulocyte colony-stimulating factor (G-CSF) when a chitosan-benzoic acid conjugate prepared from conjugation with 20 mM benzoic acid was used;
Fig. 6 shows a pharmacokinetic pattern of G-CSF when a chitosan-benzoic acid conjugate prepared from conjugation with 25 mM benzoic acid (red curve) or a chitosan-benzoic acid conjugate prepared from conjugation with 30 mM benzoic acid (blue curve) was used;
Fig. 7 shows the relationship between the amount of a chitosan-benzoic acid conjugate prepared from conjugation with 50 mM benzoic acid and in vitro release of protein;
Fig. 8 shows an SDS-PAGE result showing the human growth hormone protein-adsorbing capacity of a chitosan-benzoic acid conjugate depending on pH (In the figure, bands 1 and 4 are the results when a chitosan-benzoic acid conjugate prepared from conjugation with 20 mM benzoic acid was used, bands 2 and 5 are the results when a chitosan-benzoic acid conjugate prepared from conjugation with 30 mM benzoic acid was used, and bands 3 and 6 are the results when a chitosan-benzoic acid conjugate prepared from conjugation with 40 mM benzoic acid was used.); and
Fig. 9 shows an SDS-PAGE result showing the BSA protein-adsorbing capacity of a chitosan-benzoic acid conjugate depending on pH (In the figure, bands 1 and 4 are the results when a chitosan-benzoic acid conjugate prepared from conjugation with 20 mM benzoic acid was used, bands 2 and 5 are the results when a chitosan-benzoic acid conjugate prepared from conjugation with 30 mM benzoic acid was used, and bands 3 and 6 are the results when a chitosan-benzoic acid conjugate prepared from conjugation with 40 mM benzoic acid was used.).

### Best Mode

Hereinafter, the present disclosure will be described in more detail through examples. The following examples are for illustrative purposes only. Those skilled in the art will understand that the scope of the present disclosure is not limited by the examples.

### Example 1: Preparation of chitosan-benzoic acid conjugate

Chitosan was used as a biocompatible polymeric material. The used chitosan was a water-soluble chitosan having a degree of deacetylation of 84.5% and a molecular weight of 20-50 kD (Mirae Biotech, Korea). A 0.2% water-soluble chitosan solution prepared by dissolving in distilled water for injection was put in containers A and B, 40 mL each. Then, a 10% benzoic acid (Sigma) solution was added to the containers A and B to final concentrations of 15 mM and 30 mM, respectively. In order to induce covalent bonding between the amino group of the chitosan polymer chain and the added benzoic acid, 1-ethyl-3-(3-dimethylaminopropyl)carbodiimide hydrochloride (EDC, Sigma) was used. The conjugation reaction was carried out in a 10 mM MES buffer solution (pH 5.5). EDC was added to the containers A and B at concentrations of 30 mM and 50 mM, respectively, so that the benzoic acid could be conjugated enough. Before adding EDC and the MES buffer solution to each reaction container, the solution pH was adjusted to 5.0-5.5 by adding 1 mM or 5 mM hydrochloric acid or sodium hydroxide. The conjugation reaction was performed for at least 10 hours at room temperature. Immediately after the conjugation reaction was completed, the solution pH in the container A was about 6.6 and that in the container B was about 7.0. After removing the supernatant using a tabletop centrifuge, the remaining precipitate was washed with a 50% ethanol solution and centrifuged again under the same condition. After adding distilled water for injection (40 mL) to the resulting precipitate of each container, followed by sufficiently dispersing, the supernatant was removed by centrifugation and the remaining precipitate was recovered. After adding distilled water for injection (40 mL) to the resulting precipitate of each container and dispersing again, followed by addition of glacial acetic acid (50 µL) to adjust the solution pH to 4.3-4.4, the solution was kept in a refrigerator.

### Example 2: Measurement of protein-adsorbing capacity of chitosan-benzoic acid conjugate

The chitosan polymeric material conjugated with 15 mM benzoic acid prepared in Example 1 (drug delivery carrier A) was put in different containers, 2 mg (1 mL) each, and treated as described in Table 1. After centrifuging each of thus prepared solutions and collecting the precipitate, the supernatant was subjected to SDS-PAGE analysis to determine the amount of protein present in the supernatant. The protein used in this example was human growth hormone.

As a result, the drug delivery carrier A (sample A) could adsorb about 0.4 mg of protein per 2 mg of the chitosan polymer. Drug delivery carrier B (sample B), which was prepared from conjugation with 30 mM benzoic acid, showed a better protein-adsorbing capacity than the sample A. This suggests that more protein may be adsorbed as the content of the hydrophobic group conjugated to the polymer chain increases.

**Table 1**

| Drug delivery carrier A (mL) | Protein added (mg) | TPP* (mL) | Protein content in supernatant (mg) |
|---|---|---|---|
| 1 | 0.4 | 0.4 | ∼0.02 |
| 1 | 0.6 | 0.4 | ∼0.2 |
| 1 | 0.8 | 0.4 | ∼0.4 |
| 1 | 1.0 | 0.4 | ∼0.6 |
| Water, 1 mL | 0.4 | 0.4 | ∼0.4 |

| | | | |
|---|---|---|---|
| *TPP: Tripolyphosphate | | | |

### Test Example 1: Confirmation of adsorption of protein to hydrophobic group of chitosan-benzoic acid conjugate

In order to confirm that the protein-adsorbing capacity of the samples A and B originates from the chitosan polymer conjugated with the hydrophobic group benzoic acid, the protein-adsorbing capacity of a chitosan-benzoic acid conjugate was compared with that of benzoic acid-unconjugated chitosan under the same condition. The same chitosan as the one used in Example 1 was used, and the sample A prepared in Example 1 was used as the chitosan-benzoic acid conjugate. After centrifuging each of the solutions described in Table 2 and collecting the precipitate, the supernatant was subjected to SDS-PAGE analysis to determine the amount of protein present in the supernatant. The protein used in this example was human growth hormone.

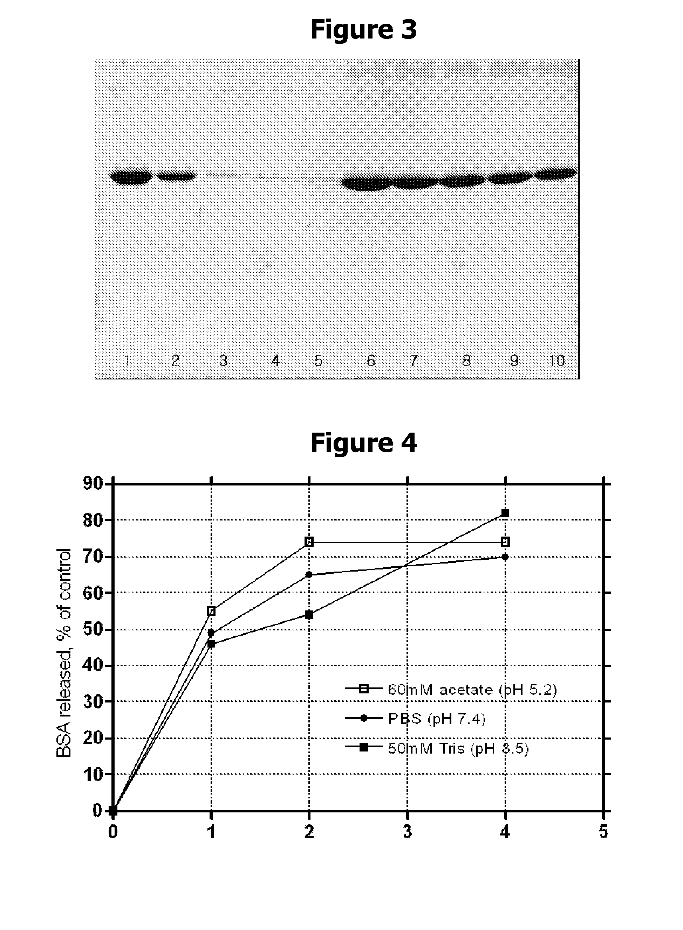

When comparing the bands 1-10 in Fig. 3, the bands 3-5 show distinct change in protein content. Thus, it was confirmed that the protein-adsorbing capacity originates from the chitosan-benzoic acid conjugate.

**Table 2**

| Band No. | Chitosan-benzoic acid conjugate (mL) | 0.2% chitosan (mL) | TPP (mL) | Protein added (mg) | Final volume (mL) |
|---|---|---|---|---|---|
| 1 | 1 | - | 0 | 0.4 | 1 |
| 2 | 1 | - | 0.1 | 0.4 | 1.1 |
| 3 | 1 | - | 0.2 | 0.4 | 1.2 |
| 4 | 1 | - | 0.3 | 0.4 | 1.3 |
| 5 | 1 | - | 0.4 | 0.4 | 1.4 |
| 6 | - | 1 | 0 | 0.4 | 1 |
| 7 | - | 1 | 0.1 | 0.4 | 1.1 |
| 8 | - | 1 | 0.2 | 0.4 | 1.2 |
| 9 | - | 1 | 0.3 | 0.4 | 1.3 |
| 10 | - | 1 | 0.4 | 0.4 | 1.4 |

### Test Example 2: Protein-adsorbing capacity of chitosan-benzoic acid conjugate depending on structural feature of group conjugated to chitosan polymer

A chitosan-benzoic acid conjugate was prepared in the same manner as Test Example 1, except for changing the group conjugated to the chitosan polymeric material from benzoic acid to 4-sulfobenzoic acid, and then protein-adsorbing capacity was tested. As a result, the chitosan polymer conjugated with 15 mM 4-sulfobenzoic acid showed no protein-adsorbing capacity at all. This reveals that the protein-adsorbing capacity is very closely related with the structural feature of the group conjugated to the chitosan polymer. That is to say, the protein-adsorbing capacity originates mainly from the hydrophobicity of the group.

### Test Example 3: Sustained release of protein from chitosan-benzoic acid conjugate

200 µg of human growth hormone and 0.4 mL of TPP (1 mg/mL) were added to 1 mL of the chitosan polymeric material conjugated with 15 mM benzoic acid prepared in Example 1, so as to adsorb the protein to the chitosan-benzoic acid conjugate. Then, through centrifugation, the conjugate was collected as precipitate. After preparing several containers filled with 5 mL of PBS and dispersing the collected precipitate in 1 mL of PBS, the resulting dispersion was added to each of the containers (1 mL per each) and mixed sufficiently. In order to investigate the release pattern of the protein adsorbed to the chitosan-benzoic acid conjugate in the PBS solution, the amount of the protein released from the conjugate into the solution was measured periodically while exposing the solution at room temperature for 6 days. The protein content was analyzed using BSA as standard and using Bradford solution as a coloring reagent. The quantified protein amount was calculated as a value relative to the amount of the initially added protein (200 µg).

The in vitro protein release test result is given in Table 3. The release amount in the PBS solution increased up to 3 days. However, from day 3, sustained release was maintained with about 60% of the initially added protein remaining adsorbed to the conjugate.

**Table 3**

| Day | (Protein content in supernatant / 200 µg) x 100 |
|---|---|
| 0 | 0 |
| 1 | 21.5 |
| 2 | 29.4 |
| 3 | 39.5 |
| 5 | 40.0 |
| 6 | 41.8 |

When a similar test was performed for about 90 hours using the chitosan polymeric material conjugated with 25 mM benzoic acid prepared in Example 1 and decreasing the amount of the initially adsorbed protein to 100 µg, 23.5% of protein was released into the solution within 1 hour and, thereafter, sustained release was maintained (see Table 4). This result reveals that, at about 90 hours, more than 75% of the initially added protein remained adsorbed to the chitosan polymeric material conjugated with 25 mM benzoic acid. Thus, it can be seen that, as the content of the conjugated benzoic acid increases, the resulting drug delivery carrier has higher hydrophobicity and, thus, the amount of released protein with time decreases greatly.

**Table 4**

| Hour | (Protein content in supernatant / 100 µg) x 100 |
|---|---|
| 0 | 0 |
| 1 | 23.5 |
| 2 | 24.3 |
| 4 | 21.2 |
| 8 | 25.9 |
| 16 | 21.2 |
| 24 | 24.3 |
| 51 | 25.9 |
| 68 | 22.8 |
| 89 | 27.8 |
| Average | 24.1 |

### Test Example 4: BSA-adsorbing capacity and release pattern of chitosan-benzoic acid conjugate

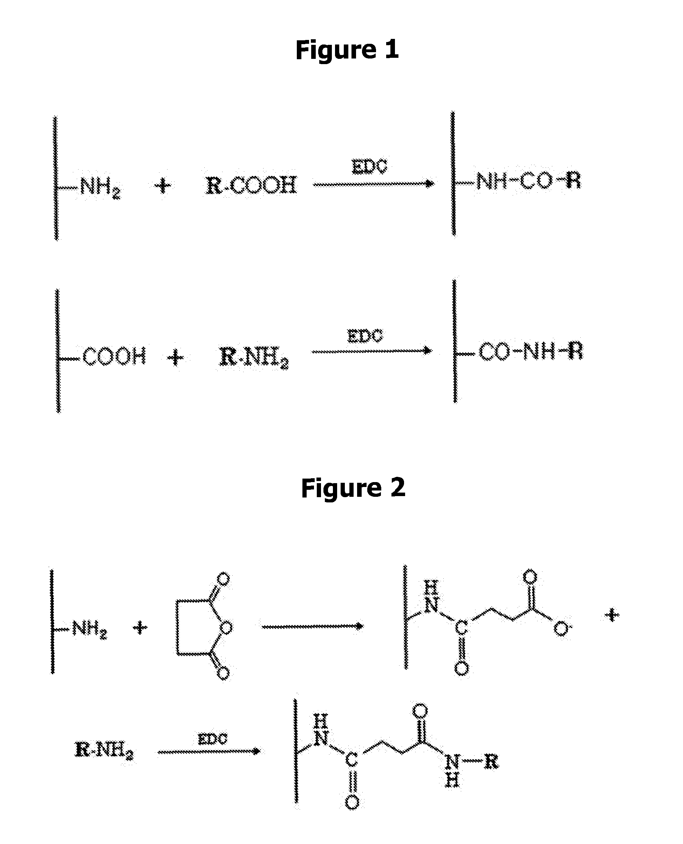

BSA-adsorbing capacity and in vitro BSA release pattern of the chitosan polymeric material conjugated with 25 mM benzoic acid used in Test Example 3 were investigated. After adding 100 µg of BSA protein to 1 mL of the precipitate to adsorb it to the drug delivery carrier, the resulting drug delivery carrier was sufficiently mixed with 5 mL of a release test solution (PBS buffer solution). In vitro release test was performed at room temperature for 4 days. The amount of the BSA protein released from the drug delivery carrier was measured by the Bradford protein assay. In order to monitor the BSA release pattern in the release test solution depending on pH, three 5 mL release test solutions were prepared using 60 mM sodium acetate (pH 5.2), PBS (pH 7.4) and 50 mM Tris (pH 8.5). As seen from the result given in Table 5, the release pattern was different depending on the pH. Initially, the release amount increased continuously. On day 2, more than 50% of protein was released from all the three solutions (Fig. 2).

**Table 5**

| Day | 60 mM sodium acetate (pH 5.2) | PBS (pH 7.4) | 50 mM Tris (pH 8.5) |
|---|---|---|---|
| 0 | 0 | 0 | 0 |
| 1 | 55 | 49 | 46 |
| 2 | 74 | 65 | 54 |
| 4 | 74 | 70 | 82 |

### Example 3: Preparation of drug delivery carrier using sodium carboxymethyl cellulose-diphenylamine conjugate or hyaluronic acid-diphenylamine conjugate

A drug delivery carrier was prepared in the same manner as Example 1 using sodium carboxymethyl cellulose, a representative anionic biocompatible polymeric material, and hyaluronic acid. Sodium carboxymethyl cellulose (25 mg) was dissolved in water and sufficiently stirred after adding diphenylamine to a final concentration of 20 mM. Then, carbodiimide (EDC) was added to a final concentration of 20 mM in an MES buffer solution (pH 5.2-5.5) with a final concentration of 50 mM. The final volume of the reaction solution was 50 mL. The mixture solution was allowed to stand at room temperature for more than 24 hours to prepare a drug delivery carrier. Upon completion of the conjugation reaction, the precipitate resulting from centrifugation was washed to obtain the drug delivery carrier. Also, another drug delivery carrier was prepared by conjugating hyaluronic acid with 50 mM diphenylamine in the same manner under the same condition.

### Test Example 5: Effect of high-pressure sterilization treatment on protein-adsorbing capacity of chitosan-benzoic acid conjugate

Drug delivery carriers in which 20 mM benzoic acid, 30 mM benzoic acid or 50 mM benzoic acid is conjugated per 50 mL of a 0.2% water-soluble chitosan solution were prepared in the same manner as Example 1. Further, a drug delivery carrier in which 50 mM benzoic acid is conjugated per 50 mL of a 0.2% chitooligosaccharide solution was prepared. Thus prepared each drug delivery carrier was sufficiently dispersed in a PBS buffer solution and, after transferring to an Eppendorf tube (1 mL per each), high-pressure steam sterilization was performed. The high-pressure sterilization treated sample was allowed to cool at room temperature and the drug delivery carrier was collected as precipitated through centrifugation. 200 µg of human growth hormone was sufficiently adsorbed by adding to the collected precipitate. Then, after centrifugation, the amount of protein present in the supernatant was measured relative to the initial addition amount. The protein adsorption was performed in PBS (pH 7.2).

As seen from the result given in Table 6, except for the chitooligosaccharide conjugate, the high-pressure sterilization treatment had no significant effect on the protein-adsorbing capacity of the drug delivery carriers.

**Table 6**

| Sample treatment | Test sample | (Protein content in supernatant / 200 µg) × 100 |
|---|---|---|
| High-pressure steam sterilization | 20 mM chitosan-benzoic acid conjugate | 16.5% |
| | 30 mM chitosan-benzoic acid conjugate | 9.5% |
| | 50 mM chitosan-benzoic acid conjugate | 1.05% |
| | 50 mM chitooligosaccharide-benzoic acid conjugate | 21% |
| Storage at room temperature | 20 mM chitosan-benzoic acid conjugate | 17.5% |
| | 30 mM chitosan-benzoic acid conjugate | 8% |
| | 50 mM chitosan-benzoic acid conjugate | 0.9% |
| | 50 mM chitooligosaccharide-benzoic acid conjugate | 2.65% |

### Test Example 6: Protein-adsorbing capacity of sodium carboxymethyl cellulose-benzylamine conjugate

A drug delivery carrier was prepared in the same manner as Example 1 by chemically conjugating 50 mL of a 0.1% sodium carboxymethyl cellulose solution with 50 mM benzylamine. Protein-adsorbing capacity of the drug delivery carrier was compared for human growth hormone and granulocyte-colony stimulating factor (G-CSF). The adsorption was induced by adding 200 µg of each protein to 1 mL of the drug delivery carrier precipitate. The adsorption and protein quantification were carried out in a PBS buffer solution (pH 7.2) for human growth hormone and in a 10 mM sodium acetate buffer solution (pH 4.0) for G-CSF. As a result, the drug delivery carrier did not absorb human growth hormone at all. In contrast, the G-CSF protein was present in the supernatant in an amount of about 4.5% of the initially added amount, meaning that about 95.5% was adsorbed to the drug delivery carrier.

### Test Example 7: In vitro release test and pharmacokinetic test of chitosan-benzoic acid conjugate

Drug delivery carriers were prepared in the same manner as Example 1 by chemically conjugating 50 mL of a 0.2% chitosan solution with 15 mM, 20 mM, 25 mM or 30 mM benzoic acid. Then, in vitro release test was carried out for G-CSF protein, and pharmacokinetic test was carried out based on the result.

### Test Example 8: In vitro release test for G-CSF protein

0.5 mL of each drug delivery carrier conjugated with 15 mM, 20 mM, 25 mM or 30 mM benzoic acid was collected as precipitate through centrifugation. After adding 200 µg of G-CSF protein to the precipitate, 10 mM sodium acetate (pH 4.0) was added to make a final volume of 0.5 mL. Each solution was dispersed uniformly and allowed to stand at room temperature for about 1-2 minutes, such that the protein could be sufficiently adsorbed to the drug delivery carrier. Then, after centrifugation, the supernatant was subjected to the quantification of protein. The same G-CSF protein as that used in the test was used as standard for the protein assay. The amount of protein present in the supernatant without being adsorbed to the drug delivery carrier was calculated relative to the initially added amount. The result is given in Table 7.

**Table 7**

| Drug delivery carrier | Non-adsorbed ratio [(protein content in supernatant / 200 µg protein) × 100] |
|---|---|
| 15 mM conjugate | ∼100% |
| 20 mM conjugate | 55.0% |
| 25 mM conjugate | 9.4% |
| 30 mM conjugate | 2.3% |

### Test Example 9: Pharmacokinetic analysis of chitosan-benzoic acid conjugate

0.5 mL of a sample containing the 20 mM benzoic acid conjugate prepared above and protein was prepared. After administering the sample to an 8-week-old SD rat via subcutaneous injection, blood was taken every day and the level of G-CSF protein in the blood was analyzed by the enzyme-linked immunosorbent assay (ELISA) method *(*Enzyme Immunoassay, E. T. Maggio, ed., CRC Press, Boca Raton, Florida, 1980; and Gaastra, W., Enzyme-linked immunosorbent assay (ELISA), in Methods in Molecular Biology, Vol. 1, Walker, J.M. ed., Humana Press, NJ, 1984). The same in vivo pharmacokinetic analysis was carried out for the 25 mM and 30 mM benzoic acid conjugates. As seen from Figs. 3 and 4, the in vivo test result was consistent with the in vitro test result. For the 25 mM and 30 mM benzoic acid conjugates, the protein release was observed on days 1 and 6.

### Test Example 10: Comparison of protein-adsorbing capacity of chitosan-benzoic acid conjugate, chitooligosaccharide-benzoic acid conjugate and mixture thereof

50 mL of a 0.2% chitosan solution and 50 mL of a 0.2% chitooligosaccharide solution were conjugated respectively with 50 mM benzoic acid in the same manner as Example 1. The following test was carried out using the resulting drug delivery carriers.

Thus prepared 1 mL of chitosan-50 mM benzoic acid conjugate (hereinafter, CTS-50) and 1 mL of chitooligosaccharide-50 mM benzoic acid conjugate (hereinafter, OCTS-50) and 2 mL of a mixture solution of the two conjugates (hereinafter, MIX-50) were prepared in plural numbers in different containers. After centrifugation, the precipitate was collected and 1 mL of 200 µg/mL BSA dissolved in PBS was added thereto and dispersed to adsorb the protein to the drug delivery carrier. Each container was kept in a refrigerator and samples were taken on days 1, 3, 5 and 7. The sample was centrifuged and the protein content in the supernatant was calculated relative to the initially added amount. The result is given in Table 8.

As a result, OCTS-50 showed better protein-adsorbing capacity than CTS-50. MIX-50 showed better protein-adsorbing capacity than CTS-50 but not better than OCTS-50. Accumulated release of protein from the drug delivery carrier with time showed gradual increase, except for OCTS-50.

**Table 8**

| Day | Accumulated release of protein in supernatant (%) | | |
|---|---|---|---|
| | OCTS-50 | MIX-50 | CTS-50 |
| 1 | 1.5 | 5.6 | 30.0 |
| 3 | 1.2 | 6.3 | 35.1 |
| 5 | 1.2 | 6.8 | 37.6 |
| 7 | 1.2 | 7.1 | 38.4 |

### Test Example 11: Relationship between total amount of drug delivery carrier and protein release

1 mL, 2 mL and 3 mL of the chitosan-50 mM benzoic acide conjugate prepared above were prepared in different containers. After centrifugation, the precipitate was collected and 1 mL of 200 µg/mL BSA dissolved in PBS was added thereto and dispersed to adsorb the protein to the drug delivery carrier. Each contained was kept in a refrigerator and samples were taken on days 1, 2, 3 and 4. The sample was centrifuged and the protein content in the supernatant was calculated relative to the initially added amount. The result is given in Table 9.

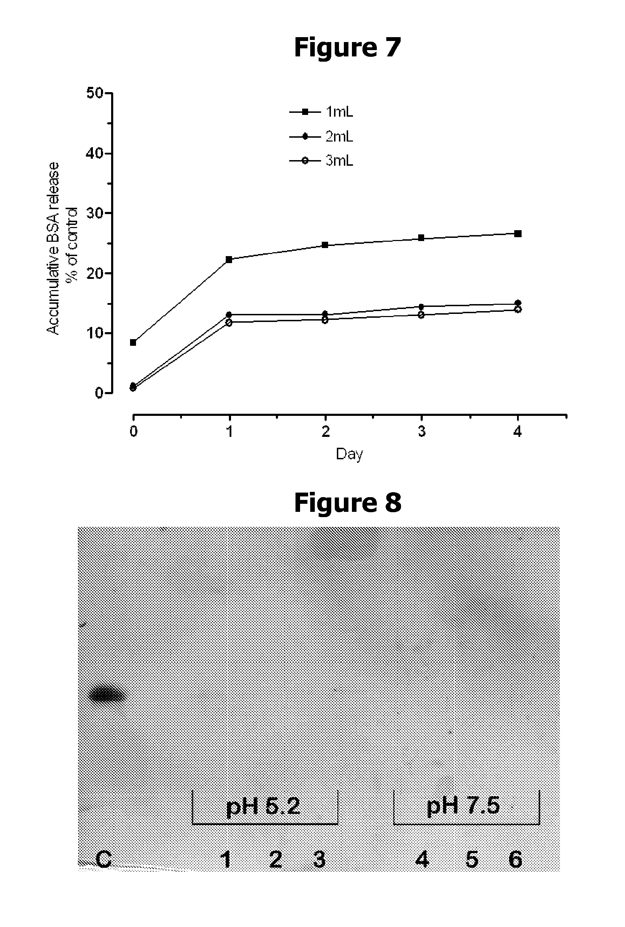

As seen from Fig. 7, the amount of released protein decreased as the total amount of the drug delivery carrier increased .

**Table 9**

| Day | 0 | 1 | 2 | 3 | 4 |
|---|---|---|---|---|---|
| 1 mL (%) | 8.5 | 22.3 | 24.7 | 25.9 | 26.6 |
| 2 mL (%) | 1.2 | 13.1 | 13.2 | 14.4 | 15.0 |
| 3 mL (%) | 0.8 | 11.8 | 12.3 | 13.1 | 14.0 |

### Example 4: Preparation of chitosan-benzylamine conjugate

Chitosan powder (100 mg) was dissolved sufficiently in a 100 mM sodium acetate buffer solution (pH 6.0) or phosphate buffer solution (pH 6.0-6.3) containing an adequate amount of ethanol or methanol. Then, an adequate amount of succinic anhydride powder was added to prepare a succinic anhydride solution having a final concentration of 50 mM or higher. After reaction at room temperature for over 24 hours while maintaining the solution pH around 6, functionally modified chitosan was recovered through dialysis. After sufficiently dialyzing with pure water, thus obtained chitosan was conjugated with 30 mM benzylamine in the same manner as Example 1 to prepare a drug delivery carrier.

### Test Example 12: Effect of pH on protein-adsorbing capacity of chitosan-benzoic acid conjugate

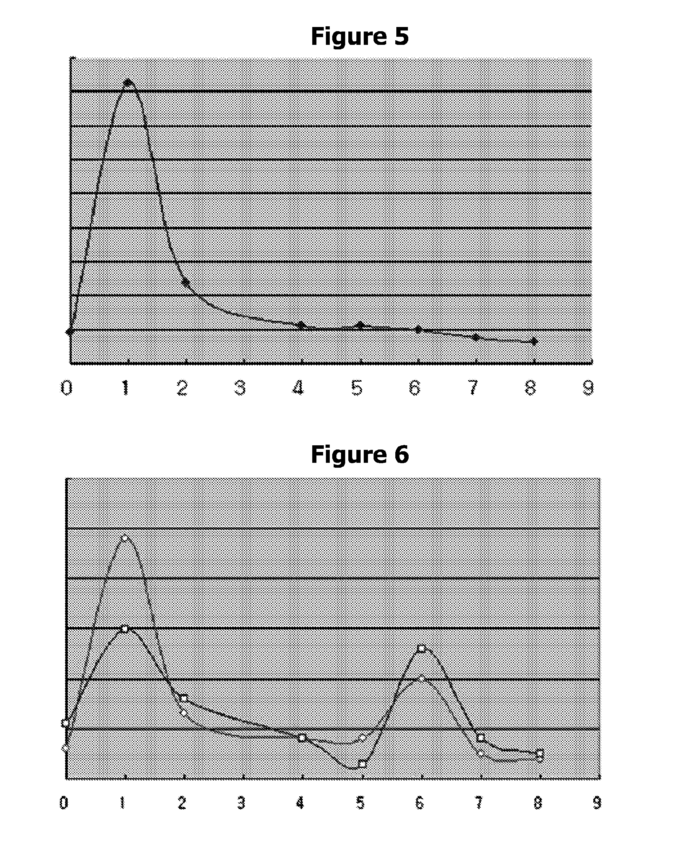

In order to find out the optimum pH condition for protein adsorption of the drug delivery carrier comprising chitosan and benzoic acid, 20 mM, 30 mM and 40 mM chitosan-benzoic acid conjugates were prepared in the same manner as Example 1. For each case, 200 µg of human growth hormone and 200 µg of BSA were adsorbed to the drug delivery carrier, based on 2 mg of chitosan, at pH 5.2 and pH 7.2, and the protein content in the supernatant was measured (Fig. 6). In this example, TPP was not treated during the protein adsorption. 20 mM sodium acetate (pH 5.2) and 20 mM Tris (pH 7.5) were used as the release test solution.

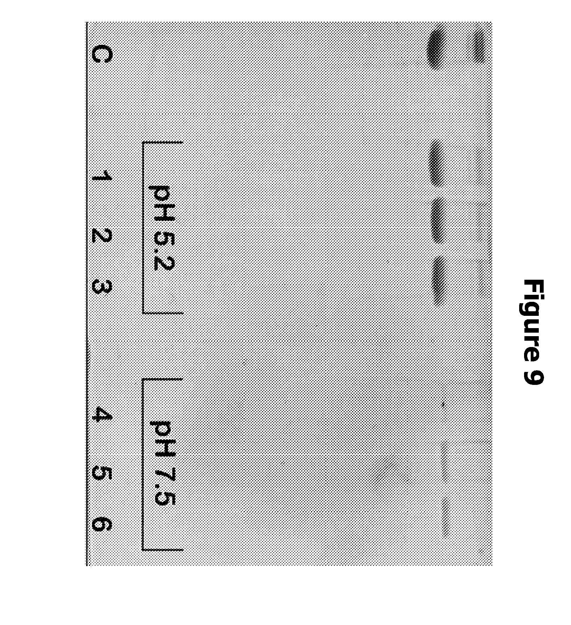

As seen from Figs. 8 and 9, for each protein, the protein-adsorbing capacity of the drug delivery carrier was different depending on the solution pH. Human growth hormone was adsorbed relatively well under acidic and weakly alkaline conditions, while BSA was not adsorbed to the drug delivery carrier under acidic conditions but was adsorbed relatively well weakly alkaline conditions.

### Test Example 13: Toxicity of chitosan-benzoic acid conjugate

Toxicity of the drug delivery carrier prepared form chemical conjugation of chitosan and benzoic acid was evaluated as follows. 5-week-old female ICR mice were grouped into groups A and B, 5 per each. 1.6 mL of the chitosan-benzoic acid conjugate prepared from conjugation with 100 mM benzoic acid in the same manner as Example 1 and obtained as precipitate from centrifugation was dispersed uniformly by adding 0.5 mL of physiological saline and subcutaneously injected to each group A mouse. Meanwhile, 3.3 mL of the chitosan-benzoic acid conjugate prepared from the same solution and obtained as precipitate from centrifugation was dispersed uniformly by adding 0.5 mL of physiological saline and subcutaneously injected to each group B mouse. The body weight change of the mice was monitored for 7 days. The result is shown in Table 10. Neither the group A (137 mg chitosan/kg BW, 835 mg benzoic acid/kg BW, total 972 mg solid/kg BW) nor the group B (282 mg chitosan/kg BW, 1,723 mg benzoic acid/kg BW; total 2,005 mg solid/kg BW) showed significant difference from the control group. For reference, a normal 5-week-old female ICR mouse weighs 21-25 g, and a normal 6-week-old female ICR mouse weighs 23-28 g. Thus, it was revealed that the drug delivery carrier according to the present disclosure has little toxicity.

**Table 10**

| Day | Average BW immediately before administration (g) | Average BW on day 1 (g) | Average BW on day 2 (g) | Average BW on day 7 (g) |
|---|---|---|---|---|
| Group A | 23.36 | 23.78 | 25.08 | 26.66 |
| Standard deviation | 1.4 | 1.3 | 1.4 | 1.7 |
| % of control | 100.0% | 101.8% | 107.4% | 114.1% |
| Group A | 21.74 | 22.04 | 23.26 | 25.34 |
| Standard deviation | 0.6 | 0.7 | 0.6 | 1.3 |
| % of control | 100.0% | 101.4% | 107.0% | 116.6% |

### Test Example 14: Hydrophobic material-adsorbing capacity of chitosan-benzoic acid conjugate

The adsorbing capacity of the 15 mM drug delivery carrier prepared in Example 1 for BBR-250 (Brilliant Blue R-250), the typical synthetic hydrophobic material, was evaluated. BBR-250 was dissolved in ethanol and adsorbing capacity was evaluated in the concentration range of 2.4 to 120 µM. 1 mL of 120 µM/mL BBR-250 was added to chitosan unconjugated with benzoic acid (2 mg based on chitosan) or the 15 mM chitosan-benzoic acid conjugate (2 mg based on chitosan), respectively, to adsorb BBR-250 to the drug delivery carrier. After collecting each drug delivery carrier through centrifugation, the content of BBR-250 present in the supernatant was measured. As a result, the supernatant of the sample treated with chitosan showed a BBR-250 content of 81 µM/mL, whereas the supernatant of the sample treated with the chitosan-benzoic acid conjugate showed a BBR-250 content of 7.2 µM/mL. Thus, it was confirmed that the drug delivery carrier according to the present disclosure adsorbs BBR-250 well, which is a typical low-molecular-weight hydrophobic substance.

The features and advantages of the present disclosure may be summarized as follows.

The drug delivery carrier according to the present disclosure having the hydrophobic group conjugated to the biocompatible polymer may be useful for adsorption of synthetic drugs having very low solubility in water. Further, it may regulate discharge rate of adsorbed drugs by regulating a portion of hydrophobic groups conjugated to the polymeric material. Thus, the present disclosure provides a broad-spectrum platform technology applicable to new hydrophobic synthetic drugs to be developed in the future as well as those that have been developed already but face difficulties due to low bioavailability. The disclosed drug delivery carrier may provide considerable therapeutic convenience for patients by combining stained-release characteristics with the ability for adsorption of a hydrophobic drug having low bioavailability.

The drug delivery carrier according to the present disclosure may also be applied to protein therapeutics. For patent-expired first-generation protein drugs requiring daily or once-in-two-or-three-days injection, the present disclosure improves convenience by allowing second-generation injection formulations that are administered once a week or once or twice a month. The present disclosure provides a strong tool capable of achieving the desired effect simply by mixing with or adsorbing to the drug delivery carrier, unlike known techniques requiring modification or introduction of a special molecular structure to the first-generation or second-generation protein drug. Thus, application of the disclosed drug delivery carrier will shorten development time of next-generation protein drugs and will effectively contribute to increasing use of hydrophobic synthetic drugs. Ultimately, the disclosed drug delivery carrier will be useful for development of competitive new medicines such as sustained-release proteins and synthetic pharmaceuticals.

Those skilled in the art will appreciate that the conceptions and specific embodiments disclosed in the foregoing description may be readily utilized as a basis for modifying or designing other embodiments for carrying out the same purposes of the present disclosure. Those skilled in the art will also appreciate that such equivalent embodiments do not depart from the spirit and scope of the disclosure as set forth in the appended claims.

## Claims

1. A drug delivery carrier comprising (a) a biocompatible polymer; and (b) a hydrophobic group conjugated to the polymer.

2. The drug delivery carrier according to claim 1, wherein the biocompatible polymer is a synthetic polymer or a natural polymer.

3. The drug delivery carrier according to claim 2, wherein the synthetic polymer as the biocompatible polymer is polyester, polyhydroxyalkanoate (PHA), poly(α-hydroxy acid), poly(β-hydroxy acid), poly(3-hydroxybutyrate-co-valerate) (PHBV), poly(3-hydroxypropionate) (PHP), poly(3-hydroxyhexanoate (PHH), poly(4-hydroxy acid), poly(4-hydraxybutyrate), poly(4-hydroxyvalerate), poly(4-hydroxyhexanoate), poly(esteramide), polycaprolactone, polylactide, polyglycolide, poly(lactide-co-glycolide) (PLGA), polydioxanone, polyorthoester, polyanhydride, poly(glycolic acid-co-trimethylene carbonate), polyphosphoester, polyphosphoester urethane, poly(amino acid), polycyanoacrylate, poly(trimethylene carbonate), poly(iminocarbonate), poly(tyrosine carbonate), polycarbonate, poly(tyrosine arylate), polyalkylene oxalate, polyphosphazene, PHA-PEG, ethylene vinyl alcohol copolymer (EVOH), polyurethane, silicone, polyester, polyolefin, polyisobutylene, ethylene-α-olefin copolymer, styrene-isobutylene-styrene triblock copolymer, acryl polymer or copolymer, vinyl halide polymer or copolymer, polyvinyl chloride, polyvinyl ether, polyvinyl methyl ether, polyvinylidene halide, polyvinylidene fluoride, polyvinylidene chloride, polyfluoroalkene, polyperfluoroalkene, polyacrylonitrile, polyvinyl ketone, polyvinyl aromatic, polystyrene, polyvinyl ester, polyvinyl acetate, ethylene-methyl methacrylate copolymer, acrylonitrile-styrene copolymer, ABS resin, ethylene-vinyl acetate copolymer, polyamide, alkyd resin, polyoxymethylene, polyimide, polyether, polyacrylate, polymethacrylate or polyacrylic acid-co-maleic acid.

4. The drug delivery carrier according to claim 2, wherein the natural polymer as the biocompatible polymer is chitosan, dextran, cellulose, heparin, hyaluronic acid, alginate, inulin, starch or glycogen.

5. The drug delivery carrier according to claim 1, wherein the hydrophobic group conjugated to the polymer is an aliphatic compound having 4 or more carbon atoms or aromatic compound.

6. The drug delivery carrier according to claim 5, wherein the hydrophobic group conjugated to the polymer is an aromatic compound having 1 to 3 phenyl group(s).

7. The drug delivery carrier according to claim 5, wherein the hydrophobic group conjugated to the polymer is alkyl having 4 or more carbon atoms, alkenyl having 4 or more carbon atoms, cycloalkyl having 3 or more carbon atoms, alkoxy having 4 or more carbon atoms, aryl, carboxyaryl, aryl phosphate, arylamine, heteroaryl, arylalkyl, arylalkenyl, or alkylaryl.

8. The drug delivery carrier according to claim 7, wherein the hydrophobic group conjugated to the polymer is aryl, carboxyaryl, aryl phosphate, arylamine, heteroaryl, arylalkyl, arylalkenyl or alkylaryl.

9. The drug delivery carrier according to claim 1, wherein the biocompatible polymer has an amino group, the hydrophobic group is amide-bonded to the amino group, and the drug delivery carrier is represented by Chemical Formula I:
R₁-NH-CO-R₂ **(1)**
wherein R₁ is the backbone of the biocompatible polymer; and R₂ is aryl, heteroaryl, arylalkyl, arylalkenyl or alkylaryl.

10. The drug delivery carrier according to claim 1, wherein the biocompatible polymer has a carboxyl group, the hydrophobic group is amide-bonded to the carboxyl group, and the drug delivery carrier is represented by Chemical Formula II:
R₁-CO-NH-R₂ **(2)**
wherein R₁ is the backbone of the biocompatible polymer; and R₂ is aryl, heteroaryl, arylalkyl, arylalkenyl or alkylaryl.

11. The drug delivery carrier according to claim 1, wherein the drug delivery carrier further comprises a drug selected from a group consisting of a protein, a peptide and a non-hydrophilic chemical drug which is carried by being adsorbed to the hydrophobic group.

12. The drug delivery carrier according to claim 1, wherein the biocompatible polymer has an average molecular weight of 300,000 or less.

13. The drug delivery carrier according to claim 11, wherein the drug delivery carrier releases the drug in a sustained manner.

14. The drug delivery carrier according to claim 1, wherein 0.5-20 parts by weight of the hydrophobic group is conjugated to the biocompatible polymer based on 1 part by weight of the polymer.

15. The drug delivery carrier according to claim 1, wherein the drug delivery carrier is capable of regulating sustained release of a drug depending on: the kind of the hydrophobic group conjugated to the biocompatible polymer; the amount of the hydrophobic group conjugated to the biocompatible polymer; the content of the drug delivery carrier for adsorbing the drug; or a combination thereof.

16. The drug delivery carrier according to any one of claims 1 to 15, wherein the conjugation between the biocompatible polymer and the hydrophobic group is mediated by a linker.
